# FASCICULE DE BREVET EUROPEEN

(11) **EP 1 133 478 B1**
(45) Date de publication et mention de la délivrance du brevet: **06.01.2016**
(21) Numéro de dépôt: 99956120.2
(22) Date de dépôt: 23.11.1999
(51) Int. Cl.: C07D 239/16, C07C 279/22, C07C 311/06, A61K 31/505

(54) **NOUVEAUX DERIVES D'ACYLGUANIDINES, LEUR PROCEDE DE PREPARATION, LEUR APPLICATION COMME MEDICAMENTS ET LES COMPOSITIONS PHARMACEUTIQUES LES RENFERMANT**
ACYLGUANIDINEN DERIVATE, VERFAHREN ZU DEREN HERSTELLUNG, DEREN VERWENDUNG ALS HEILMITTEL UND DIESE ENTHALTENDE PHARMAZEUTISCHE ZUSAMMENSTELLUNGEN
NOVEL ACYLGUANIDINE DERIVATIVES, METHOD FOR PREPARING SAME, APPLICATION AS MEDICINES AND PHARMACEUTICAL COMPOSITIONS CONTAINING THEM

(30) Priorité: 24.11.1998 FR 9814781
(43) Date de publication de la demande: 19.09.2001
(73) Titulaire: Aventis Pharma S.A., 92160 Antony (FR); Genentech, Inc., South San Francisco, CA 94080-4990 (US)
(72) Inventeur: CARNIATO, Denis, 06800 Cagnes sur Mer (FR); GADEK, Thomas, R., Oakland, CA 94611 (US); GOURVEST, Jean-François, 77140 Claye-Souilly (FR); KNOLLE, Jochen, 65830 Kriftel (DE); PEYMAN, Anurschirwan, D-65779 Kelkheim (DE); RUXER, Jean-Marie, F-92130 Issy les Moulineaux (FR); BODARY, Sarah, C., San Bruno, CA 94066 (US)
(74) Mandataire: Santarelli
(86) Numéro de dépôt international: PCT/FR1999/002878
(87) Numéro de publication internationale: WO 2000/031046

(56) Documents cités:
- EP-A- 0 820 991
- WO-A-97/21726

## Description

La présente invention a pour objet de nouveaux dérivés d'acylguanidines, leur procédé de préparation, leur application comme médicaments et les compositions pharmaceutiques les renfermant.

L'invention a pour objet les composés de formule (I') : 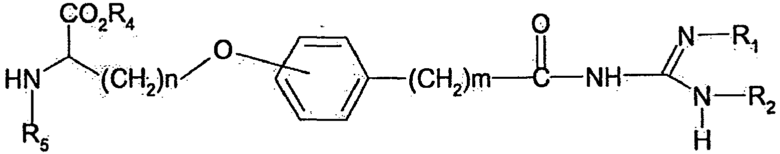 dans laquelle R₁, R₂, R₄ et R₅ ont les significations indiquées plus bas, ainsi que leur sels physiologiquement acceptables. Les composés de formule (I') sont des composés ayant une activité pharmacologique et sont donc utilisables à titre de médicaments. Ce sont des antagonistes du récepteur de la vitronectine et des inhibiteurs de l'adhésion cellulaire et ils inhibent la résorption osseuse médiée par les ostéoclastes. Ils sont donc utiles pour le traitement thérapeutique ou prophylactique de maladies qui sont causées au moins en partie par une augmentation non désirée de la résorption osseuse, par exemple l'ostéoporose. L'invention de plus a pour objet les procédés de préparation des composés de formule (I'), leur application, en particulier à titre de médicament et les compositions pharmaceutiques les renfermant.

L'os est constamment soumis à un processus dynamique qui inclut la résorption osseuse et la formation osseuse. Ces processus sont médiés via des cellules spécialisées. La formation osseuse est le résultat du dépôt d'une matrice minérale par les ostéoblastes et la résorption osseuse est le résultat de la dissolution de cette matrice osseuse par les ostéoclastes. La majorité des désordres au niveau de l'os son basés sur un équilibre perturbé entre la formation osseuse et la résorption osseuse. L'ostéoporose est caractérisée par une perte sèche de cette matrice osseuse. Un ostéoclaste mature activé résorbe l'os après adhésion à la matrice osseuse via la sécrétion d'enzyme protéolytique, et de protons à l'intérieur de la zone d'adhésion, aboutissant à des dépressions ou des creusements de la surface de l'os qui apparaissent au moment où l'ostéoclaste se détache de l'os.

Des études ont montré que la fixation de l'ostéoclaste sur l'os est médiée par des récepteurs : les intégrines. Les intégrines sont une superfamille de récepteurs médiant les processus d'adhésion cellule/cellule et plus particulièrement cellule/matrice, incluant notamment α_{IIn}β₃ comme récepteur des plaquettes sanguines (fibrinogène) et αᵥβ₃ comme récepteur de la vitronectine. Les peptides contenant le motif RGD ainsi que les anticorps anti αᵥβ₃ sont connus pour leur capacité d'inhibition de la résorption de la dentine et d'empêchement de l'adhésion des ostéoclastes sur les matrices minéralisées (Horton et al. Exp. Cell. Res. (1991), 195, 368). Le peptide Echistatine, isolé du venin de serpent contenant également un motif RGD et décrit comme inhibiteur de l'adhésion des ostéoclastes à l'os est un puissant inhibiteur de la résorption osseuse dans les tissus en culture in vitro (Sato et al. J. Cell. Biol. (1990), 111, 1713) et in vivo chez le rat (Fisher et al. Endocrinology (1993), 132, 1411).

Le récepteur αᵥβ₃ est une glycoprotéine transmembranaire qui est exprimée dans un grand nombre de cellules incluant les cellules endothéliales, les cellules du muscle lisse, l'ostéoclaste et des cellules cancéreuses ce qui entraîne ainsi une pluripotentialité des composés de formule(I') selon l'invention.

En effet, les récepteurs αᵥβ₃, exprimés au niveau de la membrane des ostéoclastes sont à la base du processus d'adhésion/résorption, contribuent à l'organisation du cytosque-lette cellulaire, et sont impliqués dans l'ostéoporose. Les récepteurs αᵥβ₃ exprimés au niveau des cellules du muscle lisse de l'aorte, stimulent leur migration vers la néointima, ce qui entraîne la formation de l'artériosclérose et la survenue de resténose post-angioplastique (Brown et al., cardiovascular Res. (1994), 28, 1815). Les cellules endothéliales secrètent des facteurs de croissance qui sont mitogènes pour l'endothélium et peuvent contribuer à la formation de nouveaux vaisseaux sanguins (Angiogenèse). Les antagonistes de l'intégrine αᵥβ₃ peuvent ainsi entraîner une régression des tumeurs cancéreuses en induisant l'apoptose des vaisseaux sanguins angiogéniques. (Brook et al. Cell (1994) 79, 1157).

Cheresh et al (Science 1995, 270, 1500) ont décrit des anticorps anti-αᵥβ₃ ou des antagonistes du récepteur αᵥβ₃ qui inhibent le processus d'angiogénèse induit par bFGF dans l'oeil de rat, une propriété qui peut être utilisée pour le traitement des rétinopathies, notamment du diabétique.

La demande de brevet WO-A-94/12181 décrit des systèmes aromatiques ou non aromatiques substitués et WO-A-94/08577 décrit des hétérocycles substitués comme antagonistes du récepteur de fibrinogène et inhibiteurs de l'agrégation plaquettaire. EP-A-528586 et EP-A-528587 décrivent des dérivés de la phénylalanine substitués par un aminoalkyle ou un hétérocycle et WO-A-95/32710 décrivent des dérivés aryliques comme inhibiteurs de la résorption osseuse par les ostéoclastes. WO-A-96/00574 décrit des benzodiazépines et WO-A-96/00730 décrit des composés inhibiteurs du récepteur fibrinogène, en particulier benzodiazépines qui sont liés à un cycle à 5 chaînons azoté comme antagonistes du récepteur de la vitronectine. DE-A-19654483 décrit des antagonistes du récepteur de la vitronectine dérivés de la tyrosine. De -A-19629816.4 revendique des dérivés de cycloalkyle comme antagonistes du récepteur de la vitronectine. D'autres investigations ont permis de montrer que les dérivés d'acylguanidine de formule(I') présentent une forte activité comme inhibiteur du récepteur de la vitronectine et de la résorption osseuse médiée via les ostéoclastes.

L'invention a pour objet les composés de formule(I') 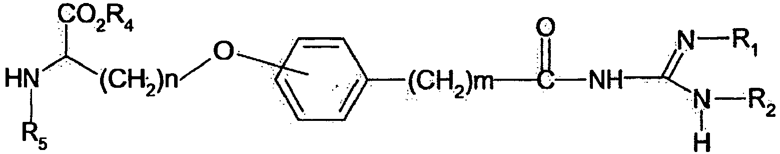 dans laquelle,
R₁ et R₂ représentent un atome d'hydrogène, ou forment ensemble un groupe -(CH₂)_{P}-, dans lequel p est 2 ou 3, ledit groupe -(CH₂)_{P}- étant non substitué ou substitué par un ou deux radicaux identiques ou différents choisis parmi halogène, (C₁-C₆)-alkyle, (C₁-C₆)-alkoxy, (C₆-C₁₄)-aryle, (C₆-C₁₄)-aryle-(C₁-C₆)alkyle, (C₅-C₁₄) -hétéroaryle, (C₅-C₁₄) -hétéroaryle- (C₁-C₆) - alkyle, (C₃-C₁₂) -cycloalkyle et (C₃-C₁₂)-cycloalkyle-(C₁-C₆)-alkyle et oxo, ledit groupe -(CH₂)_{P}- pouvant être accolé au niveau de la liaison carbone-carbone à un carbocycle ou un hétérocycle renfermant 1 ou 2 atomes d'azotes, de 5 à 7 chaînons, saturé ou insaturé, non substitué ou substitué par 1 ou 2 radicaux R₃ ;
R₃ représente un groupement alkyle ou alkyloxy renfermant de 1 à 6 atomes de carbone ;
R₄ représente un atome d'hydrogène;
R₅ représente un atome d'hydrogène, un groupement CO₂R₆, SO₂R₆, SO₂NHR₆ ou SO₂NHCO₂R₆ dans lesquels R₆ est un groupement radical (C₁-C₈)-alkyle ou naphtyle, non substitué ou substitué par R₃, ou le radical de formule (II) 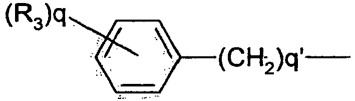 dans lequel les radicaux R₃ peuvent être identiques ou différents, et peuvent être localisés à n'importe quelle position du radical phényle, q et q' sont égaux à 0 ou 1;
m est un entier égal à 2;
n est un entier égal à 2;
le groupement acylguanidine adjacent au phényle étant en position para ou méta de l'oxygène, ainsi que leurs sels physiologiquement acceptables.

Tous les radicaux qui peuvent se retrouver plusieurs fois dans les composés de formule (I'), par exemple le radical R₃, sont indépendants les uns des autres et peuvent être identiques ou différents.

Les radicaux alkyles peuvent être linéaires ou ramifiés, saturés ou mono- ou poly-insaturés. Ceci s'applique également lorsqu'ils portent un substituant ou lorsqu'ils sont inclus dans des groupements tels que par exemple alkoxy, alkoxycarbonyle, aralkyle ou hétéroarylalkyle.
Par (C₁-C₈)-alkyle on entend les radicaux méthyle, éthyle, propyle, butyle, pentyle, hexyle, heptyle, octyle, nonyle, les n-isomères de ces radicaux, isopropyle, isobutyle, isopentyle, néopentyle, isohexyle, 3-méthylepentyle, 2,3,4-triméthylhexyle, sec-butyle, tert-butyle, tert-pentyle. Parmi les radicaux préférés on peut citer méthyle, éthyle, n-propyle, isopropyle, n-butyle, isobutyle, et tert-butyle.

Les radicaux alkylènes bivalents correspondant aux radicaux monovalents cités plus haut sont par exemple les radicaux méthylène, éthylène, 1,3-propylène, 1,2-propylène (=1-méthyléthylène), 2,3-butylène (=1,2-diméthyléthylène), 1,4-butylène, ou 1,6-hexylène.

Les radicaux alkyles insaturés sont par exemple les radicaux alkényles tels que vinyle, 1-propényle, allyle, butényle, 3-méthyle-2-butényle ou les radicaux alkynyles tels que éthynyle, 1-propynyle ou propargyle.

Par radicaux alkylène bivalents insaturés on entend les radicaux alkénylène et alkynylène qui peuvent également être linéaires ou ramifiés. Il s'agit par exemple des radicaux vinylène, propénylène, éthynylène ou propynylène.

Les radicaux cycloalkyles peuvent être monocycliques, bicycliques ou tricycliques. Il s'agit par exemple des radicaux cyclopropyle, cyclobutyle, cyclopentyle, cyclohexyle, cycloheptyle, cyclooctyle, cyclononyle, cyclodécyle, cycloundécyle, cyclododécyle, cyclotétradécyle ou cyclooctadécyle qui le cas échéant peuvent être substitués par exemple par un alkyle renfermant de 1 à 4 atomes de carbone. Comme radicaux cycloalkyles substitués, on peut citer le 4 méthylecyclo-hexyle et le 2,3-diméthylecyclohexyle.

Les radicaux bicycloalkyles et tricycloalkyles peuvent être non substitués ou substitués en toute position, par exemple par un ou plusieurs groupes oxo et/ou 1 ou plusieurs groupes alkyles identiques ou différents tels que méthyle ou isopropyle et de préférence méthyle. La liaison de jonction du radical bi ou tricyclique peut se situer en toutes positions de la molécule. La liaison peut se situer au niveau de l'atome de carbone ponté ou d'un des autres atomes de carbone. Cette liaison peut présenter également toute position du point de vue de la stéréochimie, par exemple exo ou endo. Comme exemple de radicaux bicycloalkyles ou tricycloalkyles, on peut citer le camphanyle, le bornyle, l'adamantyle tel que le 1-adamantyle ou le 2-adamantyle, le caranyle, l'épiisobornyle, l'épibornyle, le norbornyle ou le norpinanyle.

Par halogène on entend fluor, chlore, brome ou iode. Par le terme (C₅-C₁₄)-aryle on entend
- soit les radicaux (C₅-C₁₄)-aryle hétérocycliques (= (C₅-C₁₄) - hétéroaryle), dans lesquels les atomes de carbone du cycle sont remplacés par un hétéroatome tel que azote, oxygène ou soufre,
- soit les radicaux (C₆-C₁₄)-aryle carbocyclique.

Parmi les radicaux(C₆-C₁₄)-aryle carbocycliques, on peut citer le phényle, le naphtyle,le biphénylyle, l'anthryle ou le fluorényle et tout particulièrement le 1-naphtyle, le 2-naphtyle et le phényle.

Sauf indication contraire, les radicaux aryles, en particulier phényle, peuvent être non substitués ou substitués par un ou plusieurs radicaux identiques ou différents choisis parmi (C₁-C₈)-alkyle, en particulier (C₁-C₄)alkyle, (C₁-C₈)-alkoxy, halogène tel que fluor, chlore et brome, nitro, amino, trifluorométhyle, hydroxyle, méthylènedioxy, cyano, hydroxycarbonyle, aminocarbonyle, (C₁-C₄)-alkoxycarbonyle, phényle, phénoxy, benzyle et benzyloxy. En général, au plus 2 groupes nitro peuvent être utilisés dans les composés de formule(I') selon l'invention.

Dans le cas du phényle monosubstitué, le substituant peut être situé en position 2, 3 ou 4, et de préférence en position 3 ou 4. Dans le cas où le phényle est di-substitué, les substituants peuvent être en position 2, 3 ou 2, 4 ou 2, 5 ou 2, 6 ou 3, 4 ou 3, 5. De préférence, dans les phényles di-substitués, les deux substituants sont en position 3, 4. Lorsque ce phényle est tri-substitué les positions sont les suivantes : 2, 3, 4 ou 2, 3, 5 ou 2, 3, 6 ou 2, 4, 5 ou 2, 4, 6 ou 3, 4, 5. De la même manière, les radicaux naphtyles ou d'autres radicaux aryles peuvent être substitués en toute position, par exemple le radical 1-naphtyle en position 2-, 3-, 4-, 5-, 6-, 7-, et 8 et le radical 2-naphtyle en position 1-, 3-, 4-, 5-, 6-, et 7.

Le groupement (C₅-C₁₄)-aryle peut représenter également un système aromatique monocyclique ou polycyclique dans lequel 1, 2, 3, 4 ou 5 atomes de carbone du cycle sont remplacés par des hétéroatomes, en particulier identiques ou différents du groupe constitué de l'azote, l'oxygène et le soufre. Parmi les groupements (C₅-C₁₄)-aryle hétérocycliques (= (C₅-C₁₄)-hétéroaryle) on peut citer les groupements 2-pyridyle, 3-pyridyle, 4-pyridyle, pyrrolyle, furyle, thiényle, imidazo-lyle, pyrazolyle, oxazolyle, isoxazolyle, thiazolyle, isothiazolyle, tétrazolyle, pyridyle, pyrazinyle, pyrimi-dinyle, indolyle, isoindolyle, indazolyle, phtalazinyle, quinolyle, isoquinolyle, quinoxalinyle, quinazolinyle, cinnolinyle, β-carbolinyle, ou encore des dérivés benzo-condensés, cyclopenta-, cyclohexa-, ou cyclohepta- condensés de ces radicaux. Le système hétérocyclique peut être substitué par les mêmes substituants cités plus haut pour le système carbocyclique.

Parmi les radicaux hétéroaryles, sont préférés les systèmes aromatiques monocycliques ou bicycliques ayant 1, 2 ou 3 hétéroatomes, en particulier 1 ou 2 hétéroatomes, choisis parmi N, 0 ou S, et qui sont non substitués ou substitués par les groupes tels que (C₁-C₆)-alkyle, (C₁-C₆)-alkoxy, fluor, chlore, nitro, amino, trifluorométhyle, hydroxyle, (C₁-C₄)-alkoxycarbonyle, phényle, phénoxy, benzyloxy, et benzyle.

Tout particulièrement, on peut citer les systèmes aromatiques monocycliques ou bicyclique renfermant de 5 à 10 chaînons ayant de 1 à 3 hétéroatomes, en particulier 1 ou 2 hétéroatomes, choisis parmi N, 0 et S et qui peuvent être substitués par 1 ou 2 substituants tels que (C₁-C₄)-alkyle, (C₁-C₄)-alkoxy, phényle, phénoxy, benzyle et benzyloxy. Lorsque R₁ et R₂ forment ensemble un radical alkylène bivalent renfermant de 2 à 9 atomes de carbone, R₁ et R₂ forment ensemble, avec les deux atomes d'azote auxquels ils sont liés et l'atome de carbone central de la guanidine à laquelle les deux azotes sont liés, un 1,3-diazahétérocycle qui est lié à l'atome d'azote dans le groupement (CH₂)ₘ-CO-NH via sa position 2.

Comme exemple de 1,3-diazahétérocycles qui peuvent être substitués comme indiqués au niveau du radical (C₂-C₉)-alkylène ou de l'atome d'azote de la guanidine, on peut citer le radical 2-imidazolyle, le radical 4,5-dihydro-2-imidazolyle, le radical 1,4,5,6-tétrahydro-2-pyrimidinyle ou le radical 4,5,6,7-tétrahydro-1H-1,3-diazepin-2-yle.

Dans le cas où un cycle de 5 à 7 chaînons est condensé au niveau de la liaison carbone-carbone du radical (C₂-C₉)-alkylène, alors R₁ et R₂ forment ensemble, avec les deux atomes d'azote auquel ils sont liés et l'atome de carbone central de la guanidine à laquelle les deux azotes sont liés, un hétérocycle bicyclique qui est lié à l'atome d'azote du groupe (CH₂)ₘ-CO-NH et qui peut être substitué comme indiqué plus haut.
Les cycles de 5 à 7 chaînons condensés au niveau de la liaison carbone-carbone du radical (C₂-C₉)-alkylène peuvent être saturés, mono-insaturés, di-insaturés ou aromatiques ; il s'agit par exemple du cyclopropane, du cyclohexane, du cyclohexène, du cyclohexadiène, du cycloheptane ou du benzène

Parmi les systèmes aromatiques bicycliques liés à l'atome d'azote du groupement (CH₂)ₘ-CO-NH on peut citer le radical 1,3a,4,5,6,6a-hexahydro-1,3-diazapentalen-2-yle, le radical 1H-2-benzimidazolyle, le radical 3a,4,5,6,7,7a-hexahydro-1H-benzymidazol-2-yle, le radical 4,5,6,7-tétrahydro-1H-benzimidazol-2-yle, le radical 4,7-dihydro-1H-benzimidazole-2-yle ou le radical 1H-imidazo[4,5-b]pyridin-2-yle.

Dans le cas où le cycle condensé est substitué et/ou le radical (C₂-C₉)-alkylène est substitué, ils sont de préférence mono- ou di-substitués indépendamment l'un de l'autre par un radical R₃ identique ou différent.

Dans le cas ou R₁ et/ou R₂ sont des groupements alkyles substitués, ils sont de préférence mono- ou di-substitués indépendamment l'un de l'autre par un radical R₃ identique ou différent.

Les atomes de carbone optiquement actifs contenus dans les composés de formule (I')peuvent indépendamment les uns des autres présenter la configuration R ou la configuration S.

Les composés de formule (I') peuvent être sous la forme d'énantiomères purs ou de diastéréoisomères purs ou sous la forme d'un mélange d'énantiomères, par exemple sous forme de racémates ou de mélanges de diastéréoisomères.

Les sels physiologiquement acceptables des composés de formule (I') sont en particulier des sels pharmaceutiquement utilisables ou non toxiques ou physiologiquement utilisables.

Lorsque les composés de formule (I')renferment un groupe acide tel que l'acide carboxylique, il s'agit par exemple des sels de métaux alcalins ou alcalino terreux tels que les sels de sodium, de potassium, de magnésium, de calcium, et également les sels formés avec des ions ammonium quaternaires physiologiquement acceptables et les sels d'addition avec les acides tel que l'ammoniac et des amines organiques physiologiquement acceptables telles que par exemple la triéthylamine, l'éthanolamine ou le tris-(2-hydroxyéthyle)amine.

Lorsque les composés de formule (I')contiennent un groupe basique, ils peuvent former un sel d'addition avec les acides par exemple avec les acides inorganiques tels que l'acide chlorhydrique, sulfurique, phosphorique ou avec les acides organiques carboxyliques tels que l'acide acétique, trifluoroacétique, citrique, benzoique, maléique, fumarique, tartarique, methanesulfonique ou para toluène sulfonique.

Les composés de formule (I')qui comportent un groupe basique et un groupe acide, tel que par exemple guanidino et carboxylique, peuvent être présents sous forme de Zwiterions (bétaînes), qui sont également inclus dans la présente invention.
L'anion Q⁻ physiologiquement acceptable qui est contenu dans les composés de formule (I') lorsque R₄ est un radical alkyle substitué par un groupement ammonium chargé, est de préférence un anion monovalent ou un équivalent d'anion polyvalent d'un acide organique ou inorganique non toxique, physiologiquement acceptable et en particulier pharmaceutiquement acceptable, par exemple l'anion ou un équivalent d'anion d'un des acides cités plus haut utile pour la formation des sels d'addition. Q⁻ peut être par exemple un des anions (ou équivalent d'anion) d'un groupe choisi parmi chlore, sulfate, phosphate, acétate, trifluoroacétate, citrate, benzoate, maléate, fumarate, tartrate, méthanesulfonate et paratoluènesulfonate.

Les sels des composés de formule (I') peuvent être obtenus par les méthodes ordinaires connues de l'homme du métier, par exemple en combinant un composé de formule (I') avec un acide organique ou inorganique ou une base dans un solvant ou un dispersant ou à partir d'un autre sel par échange de cation ou d'anion.

L'invention inclut également tous les sels des composés de formule (I') qui, à cause de leur faible acceptabilité physiologique, ne sont pas directement utilisable comme médicament, mais sont utilisables comme produits intermédiaires pour mettre en oeuvre des modifications chimiques ultérieures au niveau des composés de formule (I') ou comme produits de départ pour la préparation de sels physiologiquement acceptables.

La présente invention inclut également tous les solvates des composés de formule (I') par exemples les hydrates, les solvates formés avec les alcools, et tous les dérivés des composés de formule (I') par exemple les esters, et autres dérivés physiologiquement acceptables, ainsi que les métabolites des composés de formule (I').

Parmi les composés préférés de formule (I'), se trouvent les composés dans lesquels le carbone asymétrique portant les groupements CO₂R₄ et NHR₅ est de configuration S.

L'invention a tout particulièrement pour objet les composés de formule (I') tels que définis plus haut dans lesquelles R₅ est un radical CO₂R₆, R₆ étant tel que défini plus haut et notamment -CH₂Ph, -C(CH₃)₃, le groupement acylguanidine adjacent au phényle étant en position para ou méta de l'oxygène, ainsi que leurs sels physiologiquement acceptables.

L'invention a tout particulièrement pour objet les composés de formule (I') telles que définies plus haut dans lesquelles R₅ est un radical SO₂R₆, R₆ étant tel que défini plus haut et notamment alkyle renfermant de 1 à 6 atomes de carbone, naphtyle et phényle substitué par un ou plusieurs radicaux alkyle renfermant de 1 à 6 atomes de carbone, le groupement acylguanidine adjacent au phényle étant en position para ou méta de l'oxygène, ainsi que leurs sels physiologiquement acceptables.

L'invention a tout particulièrement pour objet les composés de formule (I') tels que définis plus haut dans lesquelles R₅ est un radical SO₂NHR₆ ou SO₂NHCO₂R₆, R₆ étant tel que défini plus haut et notamment -CH₂Ph, -C(CH₃)₃, le groupement acylguanidine adjacent au phényle étant en position para ou méta de l'oxygène, ainsi que leurs sels physiologiquement acceptables.

L'invention a également pour objet les composés de formule (I') dont les noms suivent :
-O-[4-[3-[(aminoiminométhyl)amino]-3-oxopropyl]phényl]-N-[(phénylméthoxy)carbonyl]-homoserine ; O-[4-[3-oxo-3-[(1,4,5,6-tétrahydro-2-pyrimidinyl)-amino]-propyl]phényl]-N- [(phénylméthoxy)carbonyl]-homoserine ;
-O-[4-[3-oxo-3-[(1,4,5,6-tétrahydro-2-pyrimidinyl)amino]-propyl] phényl]-N-[(1-naphtalenyl)sulfonyl]-homoserine ;
-O-[3-[3-oxo-3-[(1,4,5,6-tétrahydro-2-pyrimidinyl)amino]-propyl]phényl]-N-[(phénylméthoxy)carbonyl]-homoserine.

La présente invention a également pour objet un procédé de préparation des composés de formule (I'). Les composés peuvent généralement être préparés, par exemple au cours d'une synthèse convergente par couplage de deux ou plusieurs fragments qui peuvent être dérivés par rétrosynthèse des composés de formule (I'). Afin d'éviter que les groupes fonctionnels puissent mener à des réactions indésirables ou secondaires au cours de chaque étape de synthèse, il peut être avantageux ou nécessaire au cours de la synthèse des composés de formule (I'), d'introduire les groupes fonctionnels sous forme de précurseurs qui sont par la suite convertis en groupes fonctionnels désirés ou de bloquer temporairement ces groupes fonctionnels en mettant en oeuvre une stratégie de groupe protecteur appropriée à la synthèse qui est connue par l'homme de l'art (Greene, Wuts protective Group in Organic Synthesis, Wiley 1991).

Ainsi les composés de formule (I') peuvent être préparés, par exemple, en couplant un acide carboxylique ou un dérivé d'acide carboxylique de formule (III) 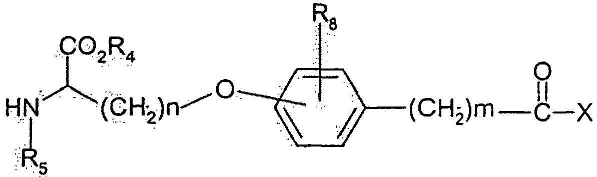 dans laquelle R₄, R₅, n et m sont tels que définis plus haut pour la formule (I'), X est un groupe partant substituable par un nucléophile, et où, le cas échéant, les groupes fonctionnels sont sous forme de précurseurs ou sous forme protégée, avec une guanidine ou un dérivé de la guanidine de formule (IV) 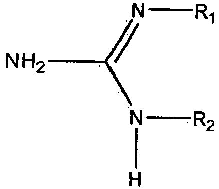 dans laquelle R₁ et R₂ sont tels que définis plus haut dans la formule (I'), et où, le cas échéant, les groupes fonctionnels sont sous forme de précurseurs ou sous forme protégée, lesdits groupes fonctionnels éventuellement présents sous forme de précurseur ou sous forme protégée, étant par la suite convertis en groupes présents dans les composés de formule (I').

Le groupement COX dans la formule (III) est de préférence le groupe acide carboxylique ou un dérivé activé de l'acide carboxylique. X, par exemple est hydroxyle ou halogène, en particulier, chlore ou brome, alkoxy, de préférence méthoxy ou éthoxy, aryloxy, par exemple phénoxy, pentafluorophényloxy, phénylthio, méthylthio, 2-pyridylthio ou un hétérocycle azoté lié via un atome d'hydrogène, en particulier un azote tel que par exemple 1-imidazolyle. X peut être également par exemple (C₁-C₄)-alkyle-O-CO-O- ou tolylsulfonyloxy et le dérivé d'acide activé peut être un anhydride mixte.

Si X est un hydroxyle, donc si la guanidine de formule (IV) réagit avec un acide carboxylique de formule (III), alors l'acide carboxylique est d'abord activé. L'activation peut être effectuée par exemple avec le dicyclohexylcarbodiimide (DCCI) ou avec le O-((cyano(éthoxycarbonyl)-métylène)amino)-1,1,3,3-tétraméthyluronium tétrafluoroborate (TOTU; König et al, Proc. 21st Europ. Peptide Symp. 1990 (Eds Giralt, Andreu), Escom, Leiden 1991, p.243) ou d'autres agents activants courants en synthèse peptidique. Outre les guanidines libres de formule (IV), les sels de guanidine peuvent également être mis en oeuvre dans la réaction avec les composés de formule (III), les guanidines libres étant formés insitu ou par une étape séparée au moyen d'une base.

La réaction d'un dérivé activé d'acide carboxylique de formule (III) avec la guanidine (ou dérivé) de formule (IV) est de préférence effectuée d'une manière connue en soi dans un solvant organique protique ou aprotique, mais inerte. Dans ce cas on utilise des solvants tels que le méthanol, l'isopropanol, le tert-butanol, le diméthylformamide ou le tétrahydrofurane à des températures allant de 0 C à la température de reflux de ces solvants, notamment lors de la réaction des esters méthyliques ou éthyliques (X est un méthoxy ou un éthoxy) avec les guanidines.

Les réactions des composés de type COX avec les guanidines libres sont avantageusement mises en oeuvre dans un solvant aprotique inerte tel que le diméthylformamide, le tétrahydrofurane, le diméthoxyéthane, ou le dioxane, le cas échéant en additionnant une base telle que par exemple le tert-butoxide de potassium, le méthoxide de sodium ou une base organique telle que la N-méthylmorpholine. Cependant, l'eau peut également être utilisée comme solvant dans les réactions des composés de formule (III) avec les guanidines de formule (IV), par exemple en utilisant une base telle que l'hydroxyde de sodium.

Si X est le chlore, la réaction sera de préférence menée par addition d'un piégeur d'acide, par exemple d'une base ou d'un excès de guanidine (ou dérivé). Le mélange réactionnel est ensuite traité et si désiré le produit réactionnel est purifié selon les méthodes connues de l'homme du métier.

Les groupes protecteurs éventuellement présents dans les composés obtenus à partir des composés de formule (III) et (IV) sont ensuite éliminés par des procédés classiques ; par exemple, les groupes tert-butyl ester sont convertis en acide carboxylique par traitement avec de l'acide trifluoroacétique, les groupes benzyles sont éliminés par hydrogénation ou encore les groupes fluorénylméthoxycarbonyle sont éliminés en présence d'amine secondaire et d'autres réactions sont mises en oeuvre par des procédés standards, par exemple par des réactions d'acylation. Si nécessaire, la conversion en des sels physiologiquement acceptables s'effectue par des procédés connus de l'homme du métier.

Lorsque R₅ représente un atome d'hydrogène, la fonctionnalisation de l'amine en groupe présent dans les composés de formule(I'), c'est-à-dire en particulier lorsque R₅ représente un groupement CO₂R₆, SO₂R₆, SO₂NHR₆ ou SO₂NHCO₂R₆ s'effectue au niveau des composés de formules (III) ou (I') et de préférence (III). A titre d'exemple pour obtenir les composés de formule (III) avec R₅=CO₂R₆ à partir de l'amine correspondante on fait réagir un composé de formule X'-CO₂R₆, X' étant un groupe partant et notamment O-succinique ou encore un halogène. Pour obtenir les composés de formule (III) avec R₅=SO₂R₆ à partir de l'amine correspondante on fait réagir un composé de formule R₆SO₂X', X' étant notamment un halogène.
Pour obtenir les composés de formule (III) avec R₅=SO₂NHCO₂R₆ à partir de l'amine correspondante on fait réagir un composé de formule X'SO₂NHCO₂R₆, X'étant notamment un halogène, ou de préférence par action d'un isocyanate de formule CISO₂NCO en présence d'un alcool R₆-OH.
Enfin pour obtenir les composés de formule (III) avec R₅=SO₂NHR₆ à partir de l'amine correspondante on fait réagir d'abord un isocyanate du type CISO₂NCO en présence d'un alcool terbutylique, puis un halogénure de formule R₆X et enfin un agent de déprotection du groupement BOC.

Les composés de départs de formule (III) et (IV) qui sont ensuite liés pour donner les composés de formule (I') sont commerciaux, peuvent être préparés selon des procédés décrits dans la littérature ou encore sont accessibles par analogie. La préparation des composés de formule (III) est illustrée dans le schéma décrit plus bas, étant entendu que la présente invention n'est pas restreinte à ces synthèses ou ces produits de départ. Il n'y a pas de difficulté majeure pour l'homme du métier de prévoir des modifications des synthèses décrites dans notre demande pour la préparation d'autres composés de formule (I')selon l'invention. Ainsi le composé de formule (V) qui est commercial peut être condensé avec le composé de formule (VI) (décrit dans Arch. Pharm. (1995) 328, 367) pour donner un composé de formule (VII). Cette condensation peut être effectuée par exemple en présence d'une base telle que le carbonate de potassium et d'un halogénure ou tout autre milieu favorisant les substitutions nucléophiles connu de l'homme du métier. Le composé de formule (VII) est un exemple de composé de formule (III) dans laquelle X est méthoxy. On peut également effectuer cette condensation directement avec l'alcool correspondant au mésylate de formule (VI) (décrit dans Synthesis (1988),786), (cas où m=2) ou dans Chem. Lett. (1996), 8, 621-622 ( cas où m=3) en présence d'une bétaïne par la réaction de Mitsunobu

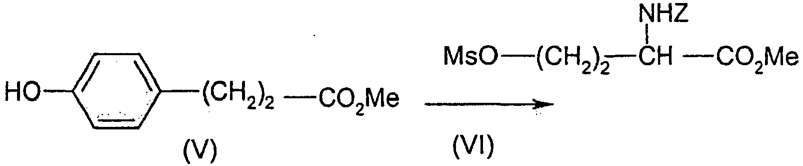

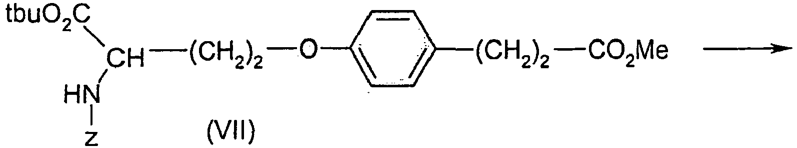

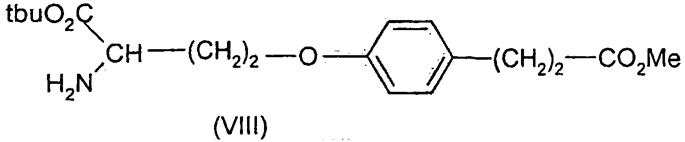

Les composés de formule (I') sont des composés ayant une activité pharmacologique et peuvent ainsi être utilisés à titre de médicaments dans le traitement ou la prévention des maladie de l'os, des maladies tumorales ainsi que des désordres cardiovasculaires.

Les composés de formule (I') ainsi que leurs sels physiologiquement acceptables peuvent être administrés aux animaux, de préférence aux mammifères et en particulier aux êtres humains comme médicaments à titre thérapeutique ou prophylactique.
Ils peuvent être administrés tels quels ou en mélange avec un ou plusieurs autres composés de formule (I') ou encore sous la forme d'une préparation pharmaceutique (composition pharmaceutique) qui permet une administration entérale ou parentérale et qui renferme à titre de composé actif une dose efficace d'au moins un composé de formule (I') et/ou ses sels physiologiquement acceptables ainsi que: des supports et/ou additifs courants et pharmaceutiquement inertes.

La présente invention a donc pour objet les composés de formule (I') et/ou leurs sels physiologiquement acceptables à titre de médicament.

La présente invention a également pour objet l'utilisation des composés de formule (I') et/ou leurs sels physiologiquement acceptables pour la préparation de médicaments destinés à la prévention ou au traitement des maladies citées plus haut ou plus bas, par exemple pour le traitement ou la prévention des maladies de l'os.

La présente invention a également pour objet les compositions pharmaceutiques qui permettent une administration entérale ou parentérale et qui, renferment à titre de composé actif une dose efficace d'au moins un composé de formule (I') et/ou ses sels physiologiquement acceptables ainsi qu'un ou plusieurs supports pharmaceutiquement inertes et le cas échéant un ou plusieurs additifs usuels.

Les médicaments peuvent être administrés oralement, par exemple sous forme de pilule, de comprimés, de comprimés enrobés, de pelliculés, de granules, de gélules et capsules molles, de solutions, de sirops, d'émulsion, de suspension ou de mélange d'aérosol.

L'administration peut cependant être effectuée par voie rectale, par exemple sous forme de suppositoire ou par voie parentérale, par exemple sous forme de solutions injectables ou d'infusions, de microcapsules ou d'implants, ou par voie percutanée, par exemple sous la forme de pommade, de solutions, de pigments ou de colorants, ou par d'autre voie telle que sous la forme d'aérosol ou de spray nasal.

Les compositions pharmaceutiques selon l'invention sont préparées selon des méthodes connues en soi, des supports organiques ou inorganiques, pharmaceutiquement inertes étant additionnés aux composés de formule (I') et/ou leurs sels physiologiquement acceptables.
Pour la production de pilule, de comprimés, de comprimés enrobés et de capsule en gélatine dure, il est possible d'utiliser par exemple, du lactose, de l'amidon de mais ou ses dérivés, du talc, de l'acide stéarique ou ses sels, etc. Les supports convenables pour des capsules en gélatine molle ou pour les suppositoires sont par exemple les graisses, les cires les polyols semi-solides ou liquides, les huiles naturelles ou modifiées etc. Les véhicules appropriés pour la préparation de solutions, par exemple les solutions injectables, les émulsions ou les sirops sont par exemple l'eau, les alcools, le glycérol, les polyols, le sucrose, les sucres invertis, le glucose, les huiles végétales, etc. Les supports convenables pour les microcapsules ou les implants sont par exemple les copolymères d'acide glyoxilique et d'acide lactique. Les préparations pharmaceutiques contiennent normalement de 0,5% à 90% en poids de composés de formule (I') et/ou leurs sels physiologiquement acceptables.

En plus des principes actifs et des supports, les préparations pharmaceutiques peuvent contenir des additifs tels que par exemple des diluants, des désintégrants, des liants, des lubrifiants, des agents mouillant, des stabilisants, des émulsifiants, des préservateurs, des agents sucrant, des colorants des agents de flaveurs ou des aromatisants, des épaississants, des agents tampons, et aussi des solvants ou des solubilisants ou des agents pour obtenir un effet retard et également des sels pour modifier la pression osmotique, des agents d'enrobage ou des antioxydants.
Elles peuvent également contenir deux ou plusieurs composés de formule (I') et/ou leurs sels physiologiquement acceptables En outre, en plus d'au moins un ou plusieurs composés de formule (I') et/ou leurs sels physiologiquement acceptables, ils peuvent contenir au moins un ou plusieurs autres principes actifs utilisables à titre thérapeutique ou prophylactique.

Les préparations pharmaceutiques (compositions pharmaceutiques) renferment normalement de 0,2 à 500 mg, et de préférence de 1 à 200 mg de composé de formule (I') et/ou leurs sels physiologiquement acceptables.

Les composés de formule (I') sont tout particulièrement des antagonistes des récepteurs de la vitronectine et sont capables ainsi par exemple d'inhiber l'adhésion des ostéoclastes sur la surface de l'os et ainsi la résorption osseuse par les ostéoclastes.

L'action des composés de formule (I') peut être démontrée par exemple dans un test dans lequel l'inhibition de la liaison de la vitronectine aux cellules qui contiennent le récepteur de la vitronectine est déterminée. Des précisions sur ce test sont données plus bas. Comme antagonistes du récepteur de la vitronectine, les composés de formule (I') et leurs sels physiologiquement acceptables et leurs prodrugs conviennent en général pour le traitement ou la prévention de maladies liées aux interactions entre les récepteurs de la vitronectine et leurs ligands, dans les processus d'interaction cellule-cellule ou cellule-matrice ou qui peuvent être influencés par l'inhibition des interactions de ce type, pour soulager ou guérir lorsqu'une inhibition des interactions de ce type est désirée. Comme on l'a expliqué au début, une telle interaction joue un rôle important dans la résorption osseuse, dans l'angiogénèse ou dans la prolifération des cellules du muscle vasculaire lisse.
Les maladies de l'os dont le traitement ou la prévention nécessitent l'emploi des composés de formule (I'), sont notamment l'ostéoporose, l'hypercalcémie, l'ostéopénie, par exemple causée par les métastases osseuses, les désordres dentaires par exemple les parodontites, l'hyperparathyroïdisme, les érosions périarticulaires dans l'arthrite rhumatoide, et la maladie de Paget. En outre les composés de formule (I') peuvent être utilisés pour soulager, empêcher ou traiter les désordres de l'os qui sont causés par les traitements, par les glucocorticoides, les thérapies liées à la prise de stéroïdes ou de corticostéroïdes ou par les déficiences d'hormones sexuelles mâles ou femelles. Tous ces désordres sont caractérisés par une perte osseuse, qui est basée par un défaut d'équilibre entre la formation osseuse et la destruction osseuse et qui peut être influencé favorablement par l'inhibition de la résorption osseuse par les ostéoclastes. A côté de cette utilisation comme inhibiteur de la résorption osseuse médiée via les ostéoclastes, les composés de formule (I') et leurs sels physiologiquement acceptables et leurs prodrugs sont utilisés comme inhibiteurs de la croissance tumorale ou des métastases cancéreuses, dans le traitement des désordres inflammatoires, pour le traitement ou la prévention des désordres cardiovasculaires, telles que l'artériosclérose ou la resténose, ou le traitement ou la prévention de néphropathie ou rétinopathie tel que par exemple la rétinopathie diabétique.

Les composés selon l'invention peuvent posséder également une activité vis-à-vis d'autres intégrines qui interagissent avec leur ligand via la séquence tripeptidique RGD (αᵥβ₁, αᵥβ₅, α_{IIb}β₃), leur conférant des propriétés pharmacologiques utilisables pour traiter les pathologies associées à ces récepteurs.
Cette activité vis-à-vis des intégrines rend ainsi les composés de formule (I') utilisables dans la prévention ou le traitement de nombreuses maladies telles que celles mentionnées plus haut ou dans la revue de Dermot Cox DN§P 8(4) Mai 1995, 197-205 dont le contenu est intégré dans la présente demande.

Lorsqu'on utilise les composés de formule (I'), les doses peuvent varier à l'intérieur de limites larges et doivent être fixées en fonction de la personne à traiter. Ceci dépend par exemple du composé employé ou de la nature et de la sévérité de la maladie à traiter et si on se trouve dans des conditions graves ou chronique ou si on met en oeuvre un traitement prophylactique.

Dans le cas d'une administration par voie orale, la dose quotidienne varie en général de 0,01 à 100 mg/kg et de préférence de 0,1 à 50 mg/kg, en particulier de 0,1 à 5 mg/kg. Par exemple pour un adulte de 75 kg on pourra envisager une dose quotidienne variant de 0,3 à 0,5 mg/kg.

Dans le cas d'une administration par voie intraveineuse, la dose quotidienne varie approximativement de 0,01 à 100 mg/kg et de préférence de 0,05 à 10 mg/kg.
La dose quotidienne peut être divisée, en particulier dans le cas de l'administration de grande quantité de principe actif, en plusieurs, par exemple 2, 3 ou 4 parts. Le cas échéant, en fonction du comportement individuel, il peut être nécessaire d'administrer les différentes doses de manière croissante ou décroissante. Mise à part l'utilisation des composés de formule (I') comme médicaments, on peut également envisager leur utilisation comme véhicule ou support de composés actifs afin de transporter ces composés actifs de manière spécifique vers un site d'action (Drug targeting, voir Targeted Drug Delivery, R.C. Juliano, Handbook of Expérimental Pharmacology, Vol 100, Ed. Born, G.V.R. et al , Springer Verlag). Les composés actifs qui peuvent être transportés sont en particulier ceux utilisés pour le traitement ou la prévention des maladies citées plus haut.

Les composés de formule (I') et leurs sels peuvent également être employés comme agent de diagnostique, par exemple pour des méthodes in vitro ou comme auxiliaire dans des études biochimiques dans lesquelles on désire bloquer le récepteur de la vitronectine ou influencer les interactions cellules-cellules ou cellules-matrices. Ils peuvent en outre être utilisés comme intermédiaire pour la préparation d'autres composés, en particulier d'autres agents actifs, qui sont accessibles à partir des composés de formule (I'), par exemple par modification ou introduction de radicaux ou de groupes fonctionnels.

### Exemples

Les produits ont été identifiés par spectre de masse (MS), infrarouge (IR) et/ou spectre RMN. Les composés, qui ont été purifiés par chromatographie en utilisant un éluant qui contient par exemple de l'acide acétique ou trifluoroacétique, et qui ensuite sont séchés ou dans lesquels lors de la dernière étape de synthèse, par exemple de l'acide trifluoroacétique a été utilisé pour éliminer un groupe protecteur tert-butyl, contiennent parfois, en fonction de la manière dont le produit a été séché, l'acide provenant de l'éluant ou de la dernière étape de synthèse et donc se trouvent partiellement ou complètement sous la forme du sel de l'acide utilisé, par exemple sous la forme d'un sel d'acide acétique ou trifluoroacétique. Ils peuvent également être plus ou moins hydratés.

### Abbrévations/noms chimiques éventuellement employés :

PCC : pyridine chlorochromate ; DMF : diméthylformamide ; THF : tétrahydrofurane ; MeOH : méthanol ; AcOEt : acétate d'éthyle ; TFA : acide trifluoroacétique ; TEA : triéthylamine ; ep. (Épaulement) ; F (fort) ; s (singulet) ; d (doublet) ; t (triplet) ; 1 (large) ; m (multiplet).

### Préparation P1

### 4-[4-[(1,1-diméthyléthoxy)]-4-oxo-3-[[(phénylméthoxy)-carbonyl]amino]butoxy]-benzènepropanoate de méthyle

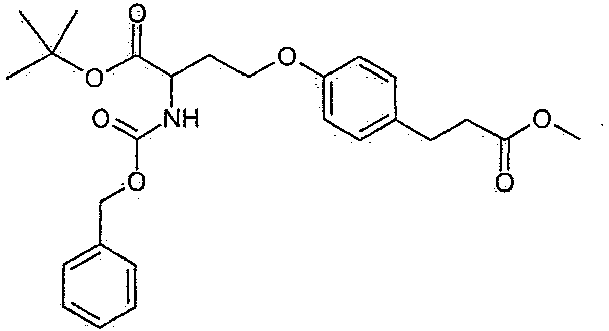

### Méthode 1

A une solution de 210 mg de 4-hydroxybenzène propanoate de méthyle et de 330 mg de carbonate de potassium dans 4 ml de THF et 4ml de DMF, on ajoute sous argon 500 mg de O-(méthyl-sulfonyle) de N-[(phénylméthoxy)carbonyl]-L-homosérinate de 1,1-diméthyléthyle préparé selon Arch. Pharm. (1995) 328, 367 dans 8 ml de THF et 8 ml de DMF, additionne quelques cristaux d'iodure de potassium et chauffe au reflux pendant environ deux heures. Après évaporation sous pression réduite, on obtient un solide blanc qui est purifié par chromatographie en éluant avec le mélange AcOEt/Cyclohexane dans un rapport 20/80, puis dans un mélange CH₂Cl₂/MeOH/NH₄OH dans le rapport 99/1/0,5. On obtient 360 mg de produit attendu.
**IR (CHCl₃)**
3430 cm⁻¹ (NH) ; 1726 cm⁻¹ (C=O) ; 1612, 1585 et 1513 cm⁻¹ (aromatique et amide II).

### Méthode 2

A une solution de 100 mg de 4-hydroxybenzène propanoate de méthyle et de 116 mg de N-[(phénylméthoxy)carbonyl]-L-homosérinate de 1,1-diméthyléthyle dans 5 ml de CH₂Cl₂ (dichlorométhane) sous argon, on ajoute en 15 minutes 223 mg de bétaïne ((T-4)-[(3,3-diméthyle)-1,2,5-thiadiazolidine-kappa.N5)1,1-dioxidato (2-)]triphényl-phosphorus) préparée selon J. Org. Chem. (1994) 59, 2289 (RN=155632-33-0). On agite 30 minutes à température ambiante puis évapore sous pression réduite. On reprend l'huile obtenue avec 10 ml de CH₂Cl₂ et 50 ml d'éther, lave la phase organique, sèche puis évapore sous pression réduite pour obtenir 330 mg de produit brut que l'on purifie par chromatographie en éluant avec le mélange AcOEt/Cyclohexane 2/8. On obtient d'une part 58 mg de produit pur et 120 mg d'une huile que l'on repurifie par chromatographie en éluant avec le mélange CH₂Cl₂/MeOH/NH₄OH 99/1/0,5. On obtient 75 mg de produit pur attendu.

### Préparation P2

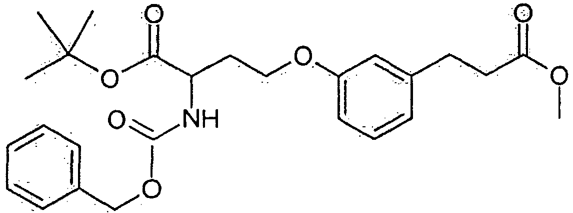

### 3-[4-[(1,1-diméthyléthoxy)]-4-oxo-3-[[(phénylméthoxy)-carbonyl]amino]butoxy]-benzènepropanoate de méthyle

### Stade A : 3-hydroxy-benzènepropanoate de méthyle

On mélange 1 g d'acide 3-hydroxy-benzènepropanoique dans 20 ml de méthanol et 8 gouttes d'acide sulfurique, porte au reflux pendant 2 heures puis évapore sous pression réduite et purifie par chromatographie en éluant avec le mélange AcOEt/Cyclohexane 50/50. On obtient 1,07 g de produit attendu.
**IR (CHCl₃) :** 3595 cm⁻¹ et associé (OH) ; 1732 cm⁻¹ (C=O de l'ester méthylique , Me : 1439 cm⁻¹) ; 1614, 1599, 1591, 1491 cm⁻¹ Aromatique.

### Stade B :

On opère comme à la préparation 1 (méthode 2) à partir de 270 mg du phénol préparé au stade A, 309 mg de N-[(phénylméthoxy)carbonyl]-L-homosérinate de 1,1-diméthyle-éthyle et 616 mg de bétaine. On obtient 320 mg de produit attendu.
**IR (CHCl₃)** Absence d'OH ; 3426 cm⁻¹ (NH) ; 1725 cm⁻¹ (C=O) 1602, 1585 et 1507 cm⁻¹ (Aromatique + Amide II)

### Exemple 1

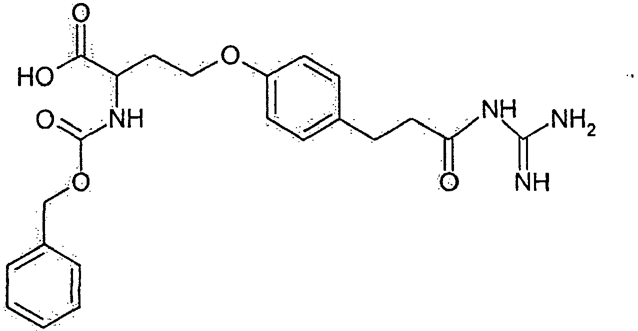

### O-[4-[3-[(aminoiminométhyl)amino]-3-oxopropyl]phényl]-N-[(phénylméthoxy)carbonyl]-homoserine

### Stade A : O-[4-[3-[(aminoiminométhyl)amino]-3-oxopropyl] phényl]-N-[(phénylméthoxy)carbonyl]-homosérinate de (1,1-diméthyl)éthyle

A une solution de 100 mg de l'ester P1 dans 2 ml de DMF, on ajoute 40 mg de guanidine base et agite à température ambiante sous argon pendant 4 heures et évapore sous pression réduite. On obtient 140 mg d'un produit brut que l'on purifie par chromatographie en éluant avec le mélange CH₂Cl₂/MeOH/AcOH/H₂O 90/10/1/1 pour obtenir 60 mg de produit pur attendu.
**IR (CHCl₃)** 3422, 3366 cm⁻¹ (NH/NH₂) ; 1717 cm⁻¹ (C=O) 1611, 1603, 1554 et 1513 cm⁻¹ (Aromatique + C=N + Amide II + NH/NH₂) Stade B : O-[4-[3-[(aminoiminométhyl)amino]-3-oxopropyl] phényl] -N-[(phénylméthoxy)carbonyl]-homoserine

A une solution de 70 mg de l'ester obtenu au stade A dans 2 ml de CH₂Cl₂, on ajoute à + 5 C, 3 ml d'acide trifluoroacétique (TFA) (3 fois 1 ml en 6 heures), agite 7 heures puis évapore sous pression réduite après addition de toluène. On ajoute ensuite 0,5 ml de méthanol et 1 ml d'acétate d'éthyle et, après filtration, coule le filtrat dans 10 ml d'éther isopropylique auquel on ajoute 2 ml de pentane. Après filtration, le solide est séché sous pression réduite et obtient 30 mg de produit attendu.
**IR nujol :** Absorption générale OH/NH ; 1722, 1705 et 1661 cm⁻¹ (C=O) ; 1613, 1569, 1540 et 1512 cm⁻¹ (C=N + Aromatique + Amide II + NH/NH₂).
**RMN (DMSO)** 2,00 (m) et 2,16 (m) 1CH₂ ; 2,99 (m) et 2,70 (m) 2CH₂ ; 3,97 (t, Ph-O-CH₂-CH₂) ; 4,06 (m, CO-CH-NHCO) ; 5,01 (s, CO₂CH₂Ph) ; 6,76 et 7,11 (AA'BB', C-Ph-O) ; 7,15 à 7,40 (m, C-Ph)

### Exemple 2

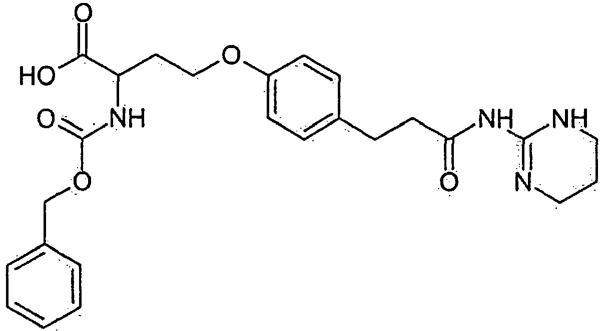

### O-[4-[3-oxo-3-[(1,4,5,6-tétrahydro-2-pyrimidinyl)amino]-propyl]phényl]-N-[(phénylméthoxy)carbonyl]-homoserine

### Stade A : O-[4-[3-oxo-3-[(1,4,5,6-tétrahydro-2-pyrimidinyl) amino] -propyl]phényl]-N-(phénylméthoxy)carbonyl]-homosérinate de (1,1-diméthyl)éthyle

On mélange sous argon 3,52 g de l'ester P1 et 1,48 g d'amino tétrahydropyrimidine base dans 35 ml de DMF, agite 5 heures à température ambiante, évapore sous pression réduite jusqu'à obtention de 5,12 g de produit brut que l'on purifie par chromatographie en éluant avec le mélange CH₂Cl₂/MeOH/AcOH/H₂O 90/10/1/1. On obtient 1,79 g de produit pur attendu.
**IR (CHCl₃)**
3428, 3264 cm⁻¹ + absorption générale (NH) ; 1716, 1685 (ép.) et 1672 cm⁻¹ (Max) (C=O) ; 1639 (ép.), 1612, 1577 et 1512 (F) cm⁻¹ (C=N +Aromatique + Amide II).

### Stade B ; O-[4-[3-oxo-3-[(1,4,5,6-tétrahydro-2-pyrimidinyl) amino]-propyl]phényl]-N-(phénylméthoxy)carbonyl]-homoserine

On opère comme à l'exemple 1 stade B à partir de 900 mg d'ester préparé au stade précédent et 12 ml de TFA dans le dichlorométhane. On obtient 750 mg de produit attendu.
**IR (CHCl₃)**
3424, 3264 cm-1 (NH), absorption générale OH/NH ; 1710, 1695 cm⁻¹ (C=O) ; 1670 cm⁻¹ (C=O + C=N) 1613, 1569 et 1512 cm⁻¹ (C=N + Aromatique + Amide II + NH/NH₂).
**RMN (CDCl₃)**
1,95 (m) et 2, 33 (ml 2 CH₂ ; 2,72 (t) et 2,86 (t) =C-CH₂-CH₂ CO ; 3,39 (m, 2=N-CH₂) ; 4,05 (m, Ph-O-CH₂-CH₂) ; 4,47 (m, CO-CH-NHCO) ; 5,11 (s, CO₂CH₂Ph) ; 5,81 (d, =C-NH-CH) ; 6,73 et 7,05 (AA'BB', C-Ph-O) ; 7,34 (s, C-Ph) ; 10,38 (sl, H mobile)

### Exemple 5

Cet exemple est présenté non comme un mode de réalisation de l'invention mais comme exemple utile à la compréhension de l'invention.

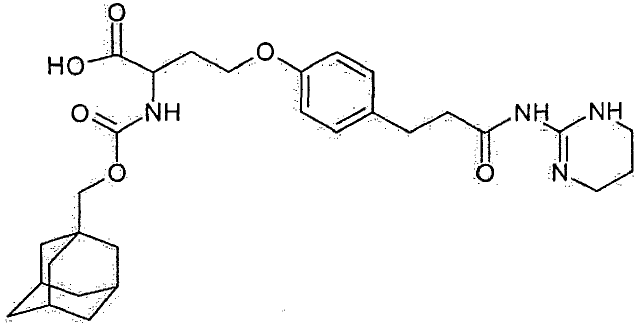

### O-[4-[3-oxo-3-[(1,4,5,6-tétrahydro-2-pyrimidinyl)amino]-propyl]phényl]-N-[[(1-tricyclo[3.3.1.1^{3,7}] décyle) -méthoxy]-carbonyl]-homoserine

### Stade A : 4-[3-(amino)-4[(1,1-diméthyléthoxy)]-4-oxobutoxy]-benzènepropanoate de méthyle

On chauffe au reflux pendant 3 heures le mélange constitué de 0,85 g d'ester P1 (préparation 1), 4,0 g de cyclohexène, 5 ml de THF, 1 ml MeOH et une quantité catalytique de Pd(OH)₂/C, puis filtre et évapore sous pression réduite le filtrat jusqu'à obtention du produit brut que l'on purifie par chromatographie en éluant avec le mélange CH₂Cl₂/MeOH 95/5. On obtient 520 mg de produit attendu.
**IR (CHCl₃)**
3390 cm⁻¹ (NH₂), 1727 cm⁻¹ (C=O), 1612, 1584 et 1513 cm⁻¹ (aromatique).

### Stade B : 4-[4-[(1,1-diméthyléthoxy)]-4-oxo-3-[[((1-tricyclo-[3.3.1.1^{3,7}] décyle)méthoxy]carbonyl]-amino]butoxy]-benzènepropanoate de méthyle

A une solution de 145 mg d'amine préparée au stade A dans 1 ml de dichlorométhane, on ajoute 65 mg de triéthylamine, refroidit à +5 C et ajoute 176 mg de 1-[[[(tricyclo-[3.3.1^{3,7}]dec-1-yl)méthoxy]carbonyl]oxy]-1H-imidazole et agite une heure à cette température puis 1 heure à température ambiante. Après évaporation sous pression réduite, on obtient 330 mg de produit brut que l'on purifie par chromatographie en éluant avec le mélange AcOEt/cyclohexane 20/80. On obtient 170 mg de produit attendu.
**IR (CHCl₃)**
3424 cm⁻¹ (NH), 1724 (large,F, C=O=) ; 1612,1512 (F) cm⁻¹ (aromatique, amide II) ; 1369, 1155 (F) cm⁻¹ tBu. Stade C : O-[4-[3-oxo-3-[(1,4,5,6-tétrahydro-2-pyrimidinyl) amino]-propyl]phényl]-N-[[(1-tricyclo[3.3.1.1^{3,7}]décyle)-méthoxy]-carbonyl]-homosérinate de (1,1-diméthyle)éthyle

On opère comme au stade A de l'exemple 2 à partir de 35 mg d'ester préparé au stade précédent et 33 mg de 1,4,5,6-tétrahydro-2-pyrimidinamine. On obtient 20 mg de produit attendu après chromatographie avec le mélange AcOEt/MeOH 80/20
MH+ = 597+
Stade D : O-[4-[3-oxo-3-[(1,4,5,6-tétrahydro-2-pyrimidinyl) amino]-propyl]phényl]-N-[[(1-tricyclo[3.3.1.1^{3,7}]décyle)-méthoxy]-carbonyl]-homoserine

On opère comme au stade B de l'exemple 1 ou 2 à partir de 85 mg d'ester préparé au stade précédent et 2 ml d'acide trifluoroacétique dans 2 ml de CH₂Cl₂. On obtient 35 mg de produit attendu.
**IR(CHCl₃)**
3424, 3264 cm⁻¹ + absorption générale (OH/NH) ; 1694, 1667 cm⁻¹ (C=O) ; 1612, 1571 et 1512 cm⁻¹ (C=N + aromatique + amide II)
**RMN (DMSO)**
1,40 à 2,20 (m) 19H Chaîne aliphatique et CH₂ centraux ; 2,59 (m) 2,77 (m) =C-CH₂-CH₂-C= ; 3,30 (m, =C-N-CH₂) ; 3,55 (s, CO₂CH₂) ; 3,97 (m, Ph-O-CH₂-CH₂) ; 4,05 (m, CO-CH-NHCO) ; 6,79 et 7,12 (AA'BB', C-Ph-O) ; 9,82 (m large) 12,50 (large) H mobiles.

### Exemple 6

Cet exemple est présenté non comme un mode de réalisation de l'invention mais comme exemple utile à la compréhension de l'invention.

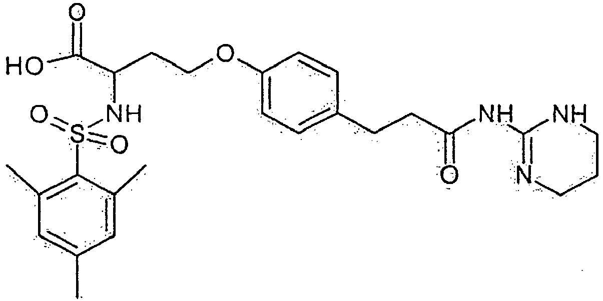

### O-[4-[3-oxo-3-[(1,4,5,6-tétrahydro-2-pyrimidinyl)amino]-propyl]phényl]-N-[(2,4,6-triméthylphényl)sulfonyl]-homoserine

### Stade A : 4-[3-[[(2,4,6-triméthylphényl)sulfonyl]amino]-4-[(1,1-diméthyléthoxy)]-4-oxobutoxy]benzènepropanoate de méthyle.

On agite pendant 3 heures à température ambiante 112 mg d'amine déprotégée préparée au stade A de l'exemple 5, 145 g de chlorure de 2-mésitylènesulfonyle, 58 mg de TEA et 1 ml de THF puis évapore sous pression réduite. On obtient 290 mg de produit brut que l'on purifie par chromatographie en éluant avec le mélange CH₂Cl₂/MeOH 99/1. On obtient 145 mg de produit pur attendu.
**IR (CHCl₃)**
3340 cm⁻¹ (NH complexe) ; 1731 cm⁻¹ (C=O) ; 1605, 1582, 1565 et 1513 cm⁻¹ (Aromatique)

### Stade B : O-[4-[3-oxo-3-[(1,4,5,6-tétrahydro-2-pyrimidinyl) amino]-propyl]phényl]-N-[(2,4,6-triméthylphényl)sulfonyl]-homosérinate de (1,1-diméthyle)éthyle

On opère comme au stade A de l'exemple 2 à partir de 133 mg de l'ester obtenu au stade précédent et 96 mg de 1,4,5,6-tétrahydro-2-pyrimidinamine dans 1 ml de DMF. On obtient 80 mg de produit pur attendu après chromatographie en éluant avec le mélange MeOH/CH₂Cl₂ 95/5
**IR (CHCl₃)**
3444, 3264 cm⁻¹ + absorption générale (NH) ; 1728 cm⁻¹ (C=O) ; 1687 et 1662 cm⁻¹ (C=O et C=N) ; 1637, 1605 (complexe) et 1512 cm⁻¹ (Système conjugué + Aromatique + amide II) Stade C : O-[4-(3-oxo-3-[(1,4,5,6-tétrahydro-2-pyrimidinyl) amino]-propyl]phényl]-N-[(2,4,6-triméthylphényl)sulfonyl]-homoserine

On opère comme au stade B de l'exemple 1 ou 2 à partir de 70 mg de l'ester obtenu au stade précédent et 1 ml de TFA dans 1,5 de CH₂Cl₂. On obtient 20 mg de produit pur attendu
**IR(Nujol)**
Absorption générale OH/NH ; 1692 cm⁻¹ (C=O) ; 1651 cm-1 (C=O + C=N) ; 1608, 1565 et 1510 cm⁻¹ (système conjugué + aromatique + amide II)
**RMN (DMSO)**
1,80 à 2,00 CH₂ centraux ; 2,75 (m) 2,50 (masqué) =C-CH₂-CH₂-C= et Ph-Me en ortho ; 2,15 (s, Ph-Me en para) ; 3,40 (m, =C-N-CH₂) ; 3,60 à 3,90 ( Ph-O-CH₂-CH₂ et CO-CH-NHCO) ; 6,65 et 7,12 (AA'BB', C-Ph-O) ; 6,86 (s,triméthylphényle) ; 7,32 (étalé) 9,82 H mobiles.

### Exemple 7

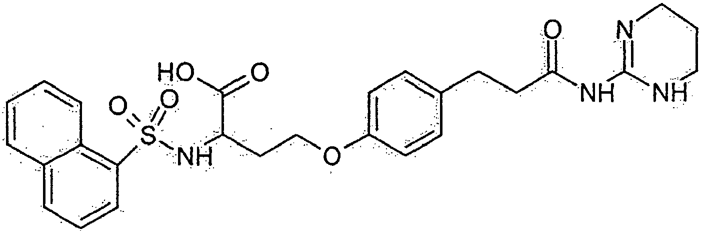

### O-[4-[3-oxo-3-[(1,4,5,6-tétrahydro-2-pyrimidinyl)amino]-propyl]phényl)-N-[(1-naphtalenyl)sulfonyl]-homoserine

On opère comme à l'exemple 6 stades A, B et C mais à partir de 90 mg d'amine préparée au stade A de l'exemple 5 et 125 mg de chlorure de naphtalène-1-sulfonyle.
**IR (CHCl₃)**
3268 cm⁻¹ + absorption générale OH/NH (NH) ; 1694 cm⁻¹ (C=O= ; 1667 cm⁻¹ (C=O + C=N) ; système conjugué + aromatique + amide II 1612, 1570 et 1513 cm⁻¹.
**RMN (CDCl₃)**
1,86 (m, CH₂ en 5') ; 3,27 (m, CH₂ en 4' et 6') ; 2,04 (m, CH₂ central de la chaîne) ; 2,56 (t, 2H) 2,75 (t, 2H) Ph-CH₂CH₂-CO ; 3,67 (m, 1H) 3,83 (m, 2H) CH₂OPh et NH-CH-CH₂ ; 6,01 (d large, NHCHCH₂) ; 6,42 et 6,89 (AA'BB', Ph-O) ; 7,35 (t, 1H) 7,48 (m, 2H) H₃ + H₆ + H₇ du naphtyle ; 7,77 (dl) 7,87 (dl) 8,14 (dd) 8,61 (d) H₂ + H₄ + H₅ + H₈ ; 10,32 (m, large) 13,71 (m, large 2H mobiles).
**MH⁺ = 539⁺**

### Exemple 8

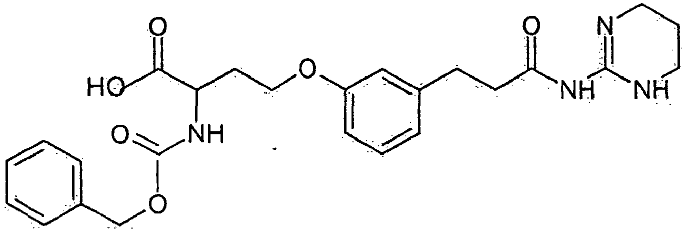

### O-[3-[3-oxo-3-[(1,4,5,6-tétrahydro-2-pyrimidinyl)amino]-propyl]phényl]-N-[(phénylméthoxy)carbonyl]-homoserine ;

On opère comme à l'exemple 2 stades A et B mais à partir de 300 mg de l'ester P2 (préparation 2) et 126 mg de 1,4,5,6-tétrahydro-2-pyrimidinamine dans le THF.
Rf (CH₂Cl₂/MeOH/AcOH/H₂O 90/10/1/1) = 0,43
**IR (CRCl₃)**
3414, 3264 cm⁻¹ + Absorption générale OH/NH (NH) ; 1754, 1709, 1693 et 1659 cm⁻¹ C=O + C=N ; 1602, 1574 et 1490 cm⁻¹ (aromatiques + amide II)
**RMN (CDCl₃)**
1,98 (m) 2,44 (m) 2CH₂ centraux ; 2,18 (m, 1H) 2,96 (m, 3H) =C-CH₂-CH₂-Ph ; 3,41 (t)2x =N-CH₂ ; 4,10 (m) 4,18 (m) Ph-O-CH₂ ; 4,34 (q 1H CO-CH-NH-CO) ; 5,11 (s, 2H, Ph-CH₂-O-CO-) ; 5,81 (d) et 5,93 (d) 1H =C-NH-CH ; 6,70 (dl, 2H, H4 et H6) ; 7,01 (s1, 1H) H1 ; 7,08 (t) H5 ; 7,36 (m, 5H Ph).

Outre les composés spécifiquement exemplifiés plus haut, d'autres composés selon l'invention sont indiqués dans le tableau ci-dessous. Ces composés sont préparés selon des méthodes décrites plus haut ou selon les variantes de celles-ci bien connues par l'homme du métier. Toutes les variables listées dans ce tableau font référence à la structure générique suivante :

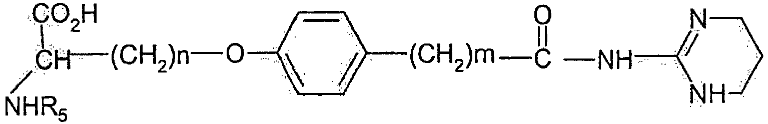

| **EXEMPLES** | **R5** | **n** | **m** |
|---|---|---|---|
| 10 | CO₂CH₂Ph | 3 | 1 |
| 11 | CO₂CH₂Ph | 2 | 3 |
| 12 | CO₂CH₂Ph | 3 | 2 |
| 13 | 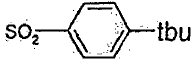 | 2 | 2 |
| 14 | SO₂nPr | 2 | 2 |
| 15 | CO₂CH₂Ph | 2 | 1 |
| 16 | SO₂CH₃ | 2 | 2 |

Les exemples 10, 11, 12 et 15 sont cités à titre de référence.

### Test pharmacologique : Test ELISA Kistrine/Récepteur Vitro-nectine (αᵥβ₃)

### Protocole :

Des plaques 96 puits MaxiSorp sont coatées une nuit à 40 C avec 100 µl de Kistrine à 1 µg/ml (dilution en tampon de coating : carbonate 0,05M / NaOH pH 9,6). Le lendemain, les puits sont vidés et les ligands (kistrine) sont ensuite fixés (tampons de fixation : PBS contenant 0,5 % de BSA (pH=7,4)) pendant 1 heure à température ambiante avec une agitation douce de 125 rpm. Les puits sont lavés six fois (tampon de lavage :PBS contenant 0,05% de Tween 20 (pH 7,7) puis on ajoute par puits et dans cet ordre :
- 40 µl de tampon d'incubation
- 10 µl de la dilution du produit à tester (les produits sont dilués dans un mélange 50:50 DMSO/Eau)
- 50 µl de récepteur αᵥβ₃ humain (cf Pytel et al. Methods Enzymol. (1987) 144 (Dilution en tampon d'incubation, à adapter suivant le lot de récepteur et selon le ligand). Le ligand, le récepteur αᵥβ₃ et les produits à étudier sont incubés pendant 3 heures à température ambiante avec une agitation douce de 125 rpm.
Les puits sont à nouveau lavés six fois, puis incubés pendant 2 heures à température ambiante avec une agitation douce de 125 rpm, en présence de 100 µl d'anticorps anti-récepteur couplé à une peroxydase (L'anticorps 4B12-HRP est dilué en tampon d'incubation (50 mM TRIS pH 7,4 ; 0,5 % BSA ; 0,05 % Tween 20 ; 1 mM MnCl₂ ; 50 µM CaCl₂ ; 50 µM MgCl₂ ; 100 mM NaCl). La dilution est à adapter suivant le lot de récepteur.

Les puits sont ensuite lavés six fois avant la mesure de la liaison ligand-récepteur faite par l'intermédiaire d'un kit révélateur de peroxydase (TBM Microwell Peroxidase Substrate System Kirkegaard ; Ref cat 50-76-00).

Ce kit contient un flacon A de substrat (3,3',5,5'-tétraméthylebenzidine à 0,4 g/l) et un flacon B (H₂O₂ à 0,02% en tampon Citrate/Acide citrique). Extemporanément, un volume de A est mélangé à un volume de B, puis le mélange réactionnel est distribué à raison de 100 µl/puits.

La réaction enzymatique se développe entre 6 à 10 minutes pour Kistrine/αᵥβ₃ puis son évolution est stoppée par l'addition de 100 µl d'acide phosphorique 1M. La densité optique est déterminée à 450 nm.

### Expression des résultats

On trace la courbe suivante : le pourcentage de liaison en fonction du logarithme de chaque concentration du produit testé.

Pour chaque produit, on détermine l'IC50 suivant la formule Suivante : IC50=(BO+ Bmin)/2

BO = Maximum de liaison en l'absence de tout produit Bmin = Minimum de liaison en présence de la concentration la plus élevée de produit.

| **EXEMPLE** | **K/VnR IC50 (µM)** |
|---|---|
| 1 | 0,031 |
| 2 | 0,0073 |
| 7 | 0,0025 |
| 8 | 0,013 |

## Revendications

1. Un composé de formule (I') 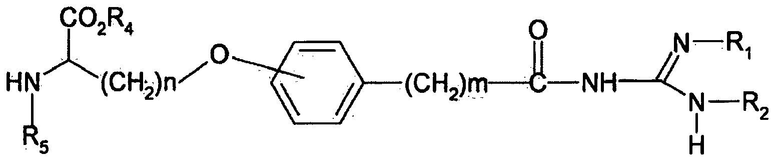 dans laquelle,
R₁ et R₂ représentent un atome d'hydrogène, ou forment ensemble un groupe -(CH₂)_{P}-, dans lequel p est 2 ou 3, ledit groupe -(CH₂)_{P}- étant non substitué ou substitué par un ou deux radicaux identiques ou différents choisis parmi halogène, (C₁-C₆)-alkyle, (C₁-C₆)-alkoxy, (C₆-C₁₄)-aryle, (C₆-C₁₄)-aryle-(C₁-C₆)alkyle, (C₅-C₁₄)-hétéroaryle, (C₅-C₁₄)-hétéroaryle-(C₁-C₆)-alkyle, (C₃-C₁₂)-cycloalkyle et (C₃-C₁₂)-cycloalkyle-(C₁-C₆)-alkyle et oxo, ledit groupe -(CH₂)_{P}- pouvant être accolé au niveau de la liaison carbone-carbone à un carbocycle ou un hétérocycle renfermant 1 ou 2 atomes d'azotes, de 5 à 7 chaînons, saturé ou insaturé, non substitué ou substitué par 1 ou 2 radicaux R₃ ;
R₃ représente un groupement alkyle ou alkyloxy renfermant de 1 à 6 atomes de carbone ;
R₄ représente un atome d'hydrogène;
R₅ représente un atome d'hydrogène, un groupement CO₂R₆, SO₂R₆, SO₂NHR₆ ou SO₂NHCO₂R₆ dans lesquels R₆ est un groupement radical (C₁-C₈)-alkyle ou naphtyle, non substitué ou substitué par R₃, ou le radical de formule (II) 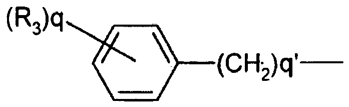 dans lequel les radicaux R₃ peuvent être identiques ou différents, et peuvent être localisés à n'importe quelle position du radical phényle, q et q' sont égaux à 0 ou 1;
m est un entier égal à 2;
n est un entier égal à 2;
le groupement acylguanidine adjacent au phényle étant en position para ou méta de l'oxygène, ainsi que leurs sels physiologiquement acceptables.

2. Un composé de formule (I') tel que défini à la revendication 1, dans laquelle R₅ est un groupement CO₂R₆, R₆ étant tel que défini à la revendication 1, le groupement acylguanidine adjacent au phényle étant en position para ou méta de l'oxygène, ainsi que leurs sels physiologiquement acceptables.

3. Un composé de formule (I') tel que défini à la revendication 1, dans laquelle R₅ est un groupement SO₂R₆, R₆ étant tel que défini à la revendication 1, le groupement acylguanidine adjacent au phényle étant en position para ou méta de l'oxygène, ainsi que leurs sels physiologiquement acceptables.

4. Un composé de formule (I') tel que défini à la revendication 1, dans laquelle R₅ est un groupement SO₂NHR₆ ou SO₂NHCO₂R₆, R₆ étant tel que défini à la revendication 1, le groupement acylguanidine adjacent au phényle étant en position para ou méta de l'oxygène, ainsi que leurs sels physiologiquement acceptables.

5. Un composé de formule (I') tel que défini à l'une quelconque des revendications 1 à 4, dont les noms suivent
O-(4-[3-[(aminoiminométhyl)amino]-3-oxopropyl]phenyl]-N-[phénylméthoxy)carbonyl]-homoserine ;
O-[4-[3-oxo-3-[(1,4,5,6-tétrahydro-2-pyrimidinyl)amino]-propyl]phényl]-N-[phénylméthoxy)-carbonyl]-homoserine ;
O-[4-[3-oxo-3-[(1,4,5,6-tétrahydro-2-pyrimidinyl)amino]-propyl]phényl]-N-[(1-naphtalenyl)sulfonyl]-homoserine ;
O-[3-[3-oxo-3-[(1,4,5,6-tétrahydro-2-pyrimidinyl)amino]-propyl]phényl]-N-[phénylméthoxy)-carbonyl]-homoserine ;
ainsi que leurs sels pharmaceutiquement acceptables.

6. Un procédé de préparation des composés de formule (I') tels que définis à l'une quelconque des revendications 1 à 5, qui comprend le couplage de deux ou plusieurs fragments qui peuvent être dérivés par rétrosynthèse des composés de formule (I').

7. Un procédé selon la revendication 6 dans lequel on fait réagir un acide carboxylique ou un dérivé d'acide carboxylique de formule (III) 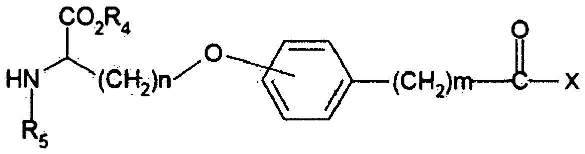 dans laquelle R₄, R₅, n et m sont tels que définis à la revendication 1 pour la formule (I'), X est un groupe partant substituable par un nucléophile, et où, le cas échéant, les groupes fonctionnels, sont sous forme de précurseur ou sous forme protégée, avec une guanidine ou un dérivé de la guanidine de formule (IV) 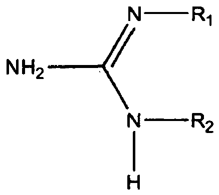 dans laquelle R₁ et R₂ sont tels que définis dans la revendication 1, et où, le cas échéant, les groupes fonctionnels éventuellement présents sous la forme de précurseurs ou sous forme protégée, étant par la suite convertis en groupes présents dans les composés de formule (I').

8. A titre de médicament, un composé de formule (I') et/ou ses sels physiologiquement acceptables tel que défini à l'une quelconque des revendications 1 à 5.

9. Une composition pharmaceutique comprenant au moins un composé de formule (I') tel que défini à l'une quelconque des revendications 1 à 5 et /ou ses sels physiologiquement acceptables ainsi qu'un ou plusieurs supports pharmaceutiquement inertes et le cas échéant un ou plusieurs additifs usuels.

10. A titre de médicament ayant une activité antagoniste du récepteur de la vitronectine, un composé de formule (I') et /ou ses sels physiologiquement acceptables tel que défini à l'une quelconque des revendications 1 à 5.

11. A titre de médicament ayant une activité inhibitrice de la résorption osseuse, un composé de formule (I') et /ou ses sels physiologiquement acceptables tel que défini à l'une quelconque des revendications 1 à 5.

12. A titre de médicament ayant une activité inhibitrice de la croissance tumorale ou des métastases cancéreuses, un composé de formule (I') et /ou ses sels physiologiquement acceptables tel que défini à l'une quelconque des revendications 1 à 5.

13. A titre de médicament ayant une activité antiinflammatoire ou pour le traitement ou la prévention des désordres cardiovasculaires, la resténose, l'artériosclérose, les néphropathies ou rétinopathies, un composé de formule (I') et/ou ses sels physiologiquement acceptables tel que défini à l'une quelconque des revendications 1 à 5.

14. Un composé de formule (I') tel que défini à la revendication 1, dans laquelle R₅ est un radical CO₂R₆ dans lequel R₆ est -CH₂Ph ou C(CH₃)₃.

15. Un composé de formule (I') tel que défini à la revendication 1, dans laquelle R₅ est un radical SO₂R₆ dans lequel R₆ est un alkyle refermant de 1 à 6 atomes de carbone, naphtyle ou phényle substitué par un ou plusieurs radicaux alkyle refermant de 1 à 6 atomes de carbone.

16. Un composé de formule (I') tel que défini à la revendication 1, dans laquelle R5 est un radical SO₂NHR₆ ou SO₂NHCO₂R₆ dans lequel R₆ est -CH₂Ph ou C(CH₃)₃.

## Patentansprüche

1. Verbindung der Formel (I'): 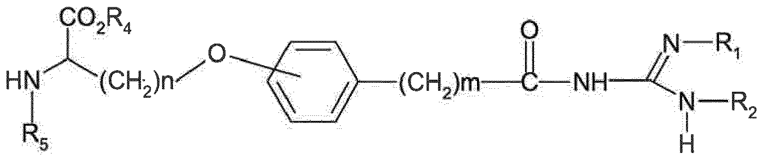 in der
R₁ und R₂ ein Wasserstoffatom bedeuten oder gemeinsam eine Gruppe -(CH₂)ₚ- bilden, in der p 2 oder 3 bedeutet, wobei die Gruppe -(CH₂)ₚ- unsubstituiert ist oder durch einen oder zwei gleiche oder verschiedene Reste, ausgewählt aus Halogen, (C₁-C₆)-Alkyl, (C₁-C₆)-Alkoxy, (C₆-C₁₄)-Aryl, (C₆-C₁₄) -Aryl- (C₁-C₆) -alkyl, (C₅-C₁₄)-Heteroaryl, (C₅-C₁₄)-Heteroaryl-(C₁-C₆)-alkyl, (C₃-C₁₂)-Cycloalkyl und (C₃-C₁₂)-Cycloalkyl-(C₁-C₆)-alkyl und Oxo, substituiert ist, wobei die Gruppe -(CH₂)ₚ- an der Kohlenstoff-Kohlenstoff-Bindung mit einem Carbocyclus oder einem Heterocyclus mit 5 bis 7 Ringgliedern, der ein oder zwei Stickstoffatome enthält, gesättigt oder ungesättigt ist und unsubstituiert oder durch ein oder zwei Reste R₃ substituiert ist, anelliert sein kann;
R₃ eine Alkyl- oder Alkyloxygruppe bedeutet, die 1 bis 6 Kohlenstoffatome enthält;
R₄ ein Wasserstoffatom bedeutet;
R₅ ein Wasserstoffatom, eine Gruppe CO₂R₆, SO₂R₆, SO₂NHR₆ oder SO₂NHCO₂R₆, in denen R₆ eine (C₁-C₈)-Alkyl- oder Naphthylrestgruppe ist, die unsubstituiert oder durch R₃ substituiert ist, oder den Rest der Formel (II) 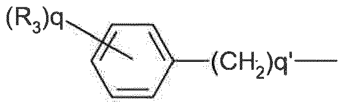 bedeutet, wobei die Reste R₃ gleich oder verschieden sein können und sich an einer beliebigen Position des Phenylrests befinden können, q und q' gleich 0 oder 1 sind;
m eine ganze Zahl gleich 2 ist;
n eine ganze Zahl gleich 2 ist;
wobei sich die neben dem Phenyl befindliche Acylguanidingruppe in para- oder meta-Position zum Sauerstoff befindet,
sowie ihre physiologisch unbedenklichen Salze.

2. Verbindung der Formel (I') wie in Anspruch 1 definiert, in der R₅ eine Gruppe CO₂R₆ bedeutet, wobei R₆ wie in Anspruch 1 definiert ist, wobei sich die neben dem Phenyl befindliche Acylguanidingruppe in para- oder meta-Position zum Sauerstoff befindet, sowie ihre physiologisch unbedenklichen Salze.

3. Verbindung der Formel (I') wie in Anspruch 1 definiert, in der R₅ eine Gruppe SO₂R₆ bedeutet, wobei R₆ wie in Anspruch 1 definiert ist, wobei sich die neben dem Phenyl befindliche Acylguanidingruppe in para- oder meta-Position zum Sauerstoff befindet, sowie ihre physiologisch unbedenklichen Salze.

4. Verbindung der Formel (I') wie in Anspruch 1 definiert, in der R₅ eine Gruppe SO₂NHR₆ oder SO₂NHCO₂R₆ bedeutet, wobei R₆ wie in Anspruch 1 definiert ist, wobei sich die neben dem Phenyl befindliche Acylguanidingruppe in para- oder meta-Position zum Sauerstoff befindet, sowie ihre physiologisch unbedenklichen Salze.

5. Verbindung der Formel (I') wie in einem der Ansprüche 1 bis 4 definiert, mit den folgenden Bezeichnungen:
O-[4-[3-[(Aminoiminomethyl)amino]-3-oxopropyl]phenyl]-N-[(phenylmethoxy)carbonyl]homoserin;
O-[4-[3-Oxo-3-[(1,4,5,6-tetrahydro-2-pyrimidinyl)amino]propyl]phenyl]-N-[(phenylmethoxy)carbonyl]homoserin;
O-[4-[3-Oxo-3-[(1,4,5,6-tetrahydro-2-pyrimidinyl)amino]propyl]phenyl]-N-[(1-naphthalenyl)sulfonyl]homoserin;
O-[3-[3-Oxo-3-[(1,4,5,6-tetrahydro-2-pyrimidinyl)amino]propyl]phenyl]-N-[(phenylmethoxy)carbonyl]homoserin;
sowie ihre pharmazeutisch unbedenklichen Salze.

6. Verfahren zur Herstellung von Verbindungen der Formel (I') wie in einem der Ansprüche 1 bis 5 definiert, das die Kopplung von zwei oder mehr Fragmenten, die sich durch Retrosynthese von Verbindungen der Formel (I') ableiten lassen, umfasst.

7. Verfahren nach Anspruch 6, bei dem man eine Carbonsäure oder ein Carbonsäurederivat der Formel (III) : 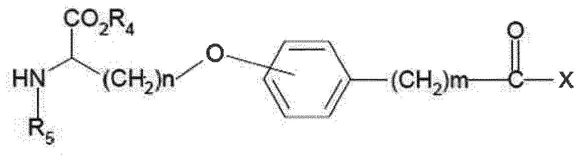 , in der R₄, R₅, n und m wie in Anspruch 1 für Formel (I') definiert sind, X eine durch ein Nucleophil substituierbare Abgangsgruppe bedeutet und wo gegebenenfalls die funktionellen Gruppen in Form von Vorläufern oder in geschützter Form vorliegen, mit einem Guanidin oder einem Guanidinderivat der Formel (IV): 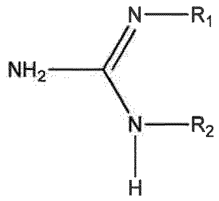 in der R₁ und R₂ wie in Anspruch 1 definiert sind und wo gegebenenfalls die funktionellen Gruppen möglicherweise in Form von Vorläufern oder in geschützter Form vorliegen, umsetzt, wobei die genannten funktionellen Gruppen anschließend in Gruppen, die in den Verbindungen der Formel (I') vorhanden sind, umgewandelt werden.

8. Verbindung der Formel (I') und/oder ihre physiologisch unbedenklichen Salze wie in einem der Ansprüche 1 bis 5 definiert als Arzneimittel.

9. Pharmazeutische Zusammensetzung, die mindestens eine Verbindung der Formel (I') wie in einem der Ansprüche 1 bis 5 definiert und/oder ihre physiologisch unbedenklichen Salze sowie einen oder mehrere pharmazeutisch inerte Träger und gegebenenfalls einen oder mehrere übliche Zusatzstoffe umfasst.

10. Verbindung der Formel (I') und/oder ihre physiologisch unbedenklichen Salze wie in einem der Ansprüche 1 bis 5 definiert als Arzneimittel mit Vitronectinrezeptorantagonistwirkung.

11. Verbindung der Formel (I') und/oder ihre physiologisch unbedenklichen Salze wie in einem der Ansprüche 1 bis 5 definiert als Arzneimittel mit knochenresorptionsinhibierender Wirkung.

12. Verbindung der Formel (I') und/oder ihre physiologisch unbedenklichen Salze wie in einem der Ansprüche 1 bis 5 definiert als Arzneimittel mit inhibierender Aktivität auf das Tumorwachstum oder von Krebsmetastasen.

13. Verbindung der Formel (I') und/oder ihre physiologisch unbedenklichen Salze wie in einem der Ansprüche 1 bis 5 definiert als Arzneimittel mit entzündungshemmender Wirkung oder für die Behandlung oder Vorbeugung von Herz-Kreislauf-Störungen, Restenose, Arteriosklerose, Nephropathien oder Retinopathien.

14. Verbindung der Formel (I') wie in Anspruch 1 definiert, in der R₅ einen Rest CO₂R₆ bedeutet, worin R₆ -CH₂Ph oder C(CH₃)₃ bedeutet.

15. Verbindung der Formel (I') wie in Anspruch 1 definiert, in der R₅ einen Rest SO₂R₆ bedeutet, worin R₆ ein Alkyl mit 1 bis 6 Kohlenstoffatomen, Naphthyl oder Phenyl, das durch einen oder mehrere Alkylreste mit 1 bis 6 Kohlenstoffatomen substituiert ist, bedeutet.

16. Verbindung der Formel (I') wie in Anspruch 1 definiert, in der R₅ einen Rest SO₂NHR₆ oder SO₂NHCO₂R₆ bedeutet, worin R₆ -CH₂Ph oder C(CH₃)₃ bedeutet.

## Claims

1. Compound of formula (I'): 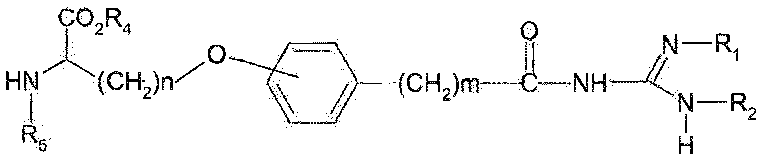 in which,
R₁ and R₂ represent a hydrogen atom, or together form a -(CH₂)ₚ- group in which p is 2 or 3, said -(CH₂)ₚ- group being unsubstituted or substituted by one or two identical or different radicals selected from halogen, (C₁-C₆)alkyl, (C₁-C₆)alkoxy, (C₆-C₁₄)aryl, (C₆-C₁₄)aryl(C₁-C₆)alkyl, (C₅-C₁₄) heteroaryl, (C₅-C₁₄) heteroaryl (C₁-C₆)alkyl, (C₃-C₁₂) cycloalkyl and (C₃-C₁₂) cycloalkyl (C₁-C₆)alkyl and oxo, it being possible for said -(CH₂)ₚ-group to be joined at the carbon-carbon bond to a saturated or unsaturated, 5- to 7-membered carbocycle or heterocycle containing 1 or 2 nitrogen atoms, which is unsubstituted or substituted by 1 or 2 R₃ radicals;
R₃ represents an alkyl or alkoxy group containing from 1 to 6 carbon atoms;
R₄ represents a hydrogen atom;
R₅ represents a hydrogen atom or a -CO₂R₆, - SO₂R₆, -SO₂NHR₆ or -SO₂NHCO₂R₆ group in which R₆ is a (C₁-C₈)alkyl or naphthyl radical group, which is unsubstituted or substituted by R₃, or the radical of formula (II): 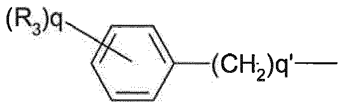 in which the R₃ radicals may be identical or different, and may be located at any position of the phenyl radical, q and q' are equal to 0 or 1;
m is an integer equal to 2;
n is an integer equal to 2;
the acylguanidine group adjacent to the phenyl being in the para or meta position with regard to the oxygen, and the physiologically acceptable salts thereof.

2. Compound of formula (I') as defined in Claim 1, in which R₅ is a CO₂R₆ group, R₆ being as defined in Claim 1, the acylguanidine group adjacent to the phenyl being in the para or meta position with regard to the oxygen, and the physiologically acceptable salts thereof.

3. Compound of formula (I') as defined in Claim 1, in which R₅ is an SO₂R₆ group, R₆ being as defined in Claim 1, the acylguanidine group adjacent to the phenyl being in the para or meta position with regard to the oxygen, and the physiologically acceptable salts thereof.

4. Compound of formula (I') as defined in Claim 1, in which R₅ is an SO₂NHR₆ or SO₂NHCO₂R₆ group, R₆ being as defined in Claim 1, the acylguanidine group adjacent to the phenyl being in the para or meta position with regard to the oxygen, and the physiologically acceptable salts thereof.

5. Compound of formula (I') as defined in any one of Claims 1 to 4, the names of which follow:
O-[4-[3-[(aminoiminomethyl)amino]-3-oxopropyl]phenyl]-N-[(phenylmethoxy)carbonyl]homoserine;
O-[4-[3-oxo-3-[(1,4,5,6-tetrahydro-2-pyrimidinyl)-amino]propyl]phenyl]-N-[(phenylmethoxy)carbonyl]-homoserine;
O-[4-[3-oxo-3-[(1,4,5,6-tetrahydro-2-pyrimidinyl)-amino]propyl]phenyl]-N-[(1-naphthalenyl)sulfonyl]-homoserine;
O-[3-[3-oxo-3-[(1,4,5,6-tetrahydro-2-pyrimidinyl)-amino]propyl]phenyl]-N-[(phenylmethoxy)carbonyl]-homoserine;
and the pharmaceutically acceptable salts thereof.

6. Process for preparing the compounds of formula (I') as defined in any one of Claims 1 to 5, which comprises the coupling of two or more fragments which can be derived by retrosynthesis from the compounds of formula (I').

7. Process according to Claim 6, in which a carboxylic acid or a carboxylic acid derivative of formula (III): 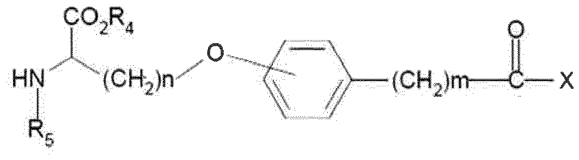 in which R₄, R₅, n and m are as defined in Claim 1 for formula (I'), X is a leaving group that can be substituted by a nucleophile, and where, if appropriate, the functional groups are in the form of precursors or in protected form, is reacted with a guanidine or a guanidine derivative of formula (IV): 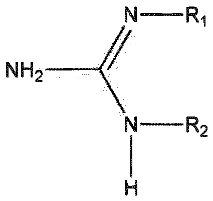 in which R₁ and R₂ are as defined in Claim 1, and where, if appropriate, the functional groups optionally present in the form of precursors or in protected form subsequently being converted to groups present in the compounds of formula (I').

8. As medicament, a compound of formula (I') and/or the physiologically acceptable salts thereof as defined in any one of Claims 1 to 5.

9. Pharmaceutical composition comprising at least one compound of formula (I') as defined in any one of Claims 1 to 5 and/or the physiologically acceptable salts thereof and also one or more pharmaceutically inert supports and, if appropriate, one or more standard additives.

10. As medicament having a vitronectin receptor antagonist activity, a compound of formula (I') and/or the physiologically acceptable salts thereof as defined in any one of Claims 1 to 5.

11. As medicament having a bone resorption inhibitory activity, a compound of formula (I') and/or the physiologically acceptable salts thereof as defined in any one of Claims 1 to 5.

12. As medicament having a tumour growth or cancer metastasis inhibitory activity, a compound of formula (I') and/or the physiologically acceptable salts thereof as defined in any one of Claims 1 to 5.

13. As medicament having an anti-inflammatory activity or for the treatment or prevention of cardiovascular disorders, restenosis, arteriosclerosis, nephropathies or retinopathies, a compound of formula (I') and/or the physiologically acceptable salts thereof as defined in any one of Claims 1 to 5.

14. Compound of formula (I') as defined in Claim 1, in which R₅ is a CO₂R₆ radical in which R₆ is -CH₂Ph or C(CH₃)₃.

15. Compound of formula (I') as defined in Claim 1, in which R₅ is an SO₂R₆ radical in which R₆ is an alkyl containing from 1 to 6 carbon atoms, naphthyl or phenyl substituted by one or more alkyl radicals containing from 1 to 6 carbon atoms.

16. Compound of formula (I') as defined in Claim 1, in which R₅ is an SO₂NHR₆ or SO₂NHCO₂R₆ radical in which R₆ is -CH₂Ph or C(CH₃)₃.
